(19) Europäisches Patentamt
European Patent Office
Office européen des brevets

(11) EP 1 742 937 B1

(12) **EUROPEAN PATENT SPECIFICATION**

(45) Date of publication and mention of the grant of the patent:
**11.11.2009 Bulletin 2009/46**

(21) Application number: **05707074.0**

(22) Date of filing: **28.01.2005**

(51) Int Cl.:
***C07D 487/04*** *(2006.01)* ***A61K 31/495*** *(2006.01)*
***A61P 35/00*** *(2006.01)*

(86) International application number:
**PCT/EP2005/000876**

(87) International publication number:
**WO 2005/075460 (18.08.2005 Gazette 2005/33)**

# (54) PYRROLOPYRIMIDINE DERIVATIVES FOR TREATING PROLIFERATIVE DISEASES

PYRROLOPYRIMIDINDERIVATE ZUR BEHANDLUNG PROLIFERATIVER KRANKHEITEN

DERIVES DE PYRROLOPYRIMIDINE SERVANT A TRAITER DES MALADIES PROLIFERATIVES

(84) Designated Contracting States:
**AT BE BG CH CY CZ DE DK EE ES FI FR GB GR HU IE IS IT LI LT LU MC NL PL PT RO SE SI SK TR**

(30) Priority: **29.01.2004 US 540034 P**

(43) Date of publication of application:
**17.01.2007 Bulletin 2007/03**

(73) Proprietors:
- **Novartis AG**
  **4056 Basel (CH)**
  Designated Contracting States:
  **BE BG CH CY CZ DE DK EE ES FI FR GB GR HU IE IS IT LI LT LU MC NL PL PT RO SE SI SK TR**
- **Novartis Pharma GmbH**
  **1230 Wien (AT)**
  Designated Contracting States:
  **AT**

(72) Inventors:
- **CARAVATTI, Giorgio**
  **CH-4103 Bottmingen (CH)**
- **TRAXLER, Peter**
  **CH-4124 Schönenbuch (CH)**
- **ESSER, Thomas**
  **Belle Mead, NJ 08502 (US)**
- **HE, Handan**
  **Morris Plains, NJ 07950 (US)**

(74) Representative: **de Weerd, Petrus G.W.**
**c/o Novartis AG, Geistiges Eigentum Konzern CH**
**4002 Basel (CH)**

(56) References cited:
**WO-A-03/013541** **WO-A-03/037897**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 742 937 B1

## Description

The invention relates to 7*H*-pyrrolo[2,3-d]pyrimidine derivatives and to processes for the preparation thereof, to pharmaceutical compositions comprising such derivatives and to the use of such derivatives - alone or in combination with one or more other pharmaceutically active compounds - for the preparation of pharmaceutical compositions for the treatment especially of a proliferative disease, such as a tumour.

The use of 7H-pyrrolo[2,3-d]pyrimidine derivatives as described, for example, in US-P 6,140,332 for the treatment of solid tumor diseases such as carcinoma of the bladder, renal carcinoma, squamous cell carcinoma of the skin, head and neck cancer, lung cancer, tumors of the gastrointestinal tract, glioma or mesothelioma is disclosed in WO 03/037897.

The present application discloses to 7*H*-pyrrolo[2,3-d]pyrimidine derivatives of formula I

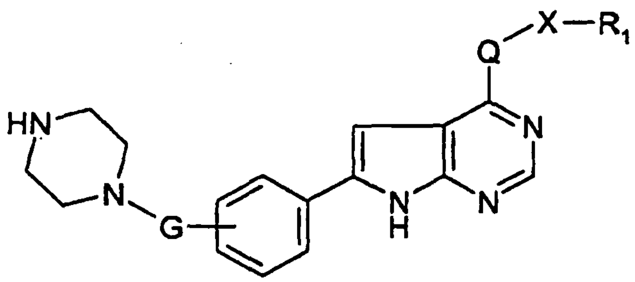

(I),

wherein

- $R_1$ is a heterocyclic radical or an unsubstituted or substituted aromatic radical;
- G is $C_1$-$C_7$-alkylene, -C(=O)-, or $C_1$-$C_6$-alkylene-C(=O)- wherein the carbonyl group is attached to the piperazine moiety;
- Q is -NH- or -O-, with the proviso that Q is -O- if G is -C(=O)- or $C_1$-$C_6$-alkylene-C(=O)-; and
- X is either not present or $C_1$-$C_7$-alkylene, with the proviso that a heterocyclic radical $R_1$ is bonded via a ring carbon atom if X is not present;

or a salt of the said compounds.

The present invention relates to a compound of formula I, wherein

- $R_1$ is phenyl, benzodioxolyl, pyridyl substituted by hydroxy or lower alkoxy having up to and including a maximum of 7 carbon atoms, indolyl substituted by halogen and lower alkyl having up to and including a maximum of 7 carbon atoms, or phenyl substituted by one or more radicals selected independently of one another from the group consisting of lower alkyl having up to and including a maximum of 7 carbon atoms, hydroxy, lower alkoxy having up to and including a maximum of 7 carbon atoms, halogen and benzyloxy;
- G is $-CH_2-$ or -C(=O)-;
- Q is -NH- or -O-, with the proviso that Q is -O- if G is -C(=O)-; and
- X is either not present, $-CH_2-$ or $-CH(CH_3)-$, with the proviso that substituted pyridyl or indolyl $R_1$ is bonded via a ring carbon atom if X is not present;

or a salt thereof.

The general terms used hereinbefore and hereinafter preferably have within the context of this disclosure the following meanings, unless otherwise indicated:

Where the plural form is used for compounds, salts, and the like, this is taken to mean also a single compound, salt, or the like.

Where compounds of formula I are mentioned which can form tautomers, it is meant to include also the tautomers of such compounds of formula I. In particular, tautomerism occurs e.g. for compounds of formula I which contain a 2-hydroxy-pyridyl radical. In such compounds the 2-hydroxy-pyridyl radical can also be present as pyrid-2(1*H*)-on-yl.

Asymmetric carbon atoms of a compound of formula I that are optionally present may exist in the (R), (S) or (R,S) configuration, preferably in the (R) or (S) configuration. Substituents at a double bond or a ring may be present in cis- (= Z-) or trans (= E-) form. The compounds may thus be present as mixtures of isomers or preferably as pure isomers.

The prefix "lower" denotes a radical having up to and including a maximum of 7, especially up to and including a maximum of 4 carbon atoms, the radicals in question being either unbranched or branched with single or multiple branching.

Lower alkyl is, for example, methyl, ethyl, n-propyl, isopropyl, n-butyl, isobutyl, sec-butyl, tert-butyl, n-pentyl, isopentyl, neopentyl, n-hexyl or n-heptyl.

Lower alkoxy is for example ethoxy or methoxy, especially methoxy.

Substituted lower alkyl is preferably lower alkyl as defined above where one or more, preferably one, substituents may be present, such as e.g. amino, N-lower alkylamino, N,N-di-lower alkylamino, N-lower alkanoylamino, N,N-di-lower alkanoylamino, hydroxy, lower alkoxy, lower alkanoyl, lower alkanoyloxy, cyano, nitro, carboxy, lower alkoxycarbonyl, carbamoyl, N-lower alkyl-carbamoyl, N,N-di-lower alkyl-carbamoyl, amidino, guanidino, ureido, mercapto, lower alkylthio, halogen or a heterocyclic radical.

A heterocyclic radical contains especially up to 20 carbon atoms and is preferably a saturated or unsaturated monocyclic radical having from 4 or 8 ring members and from 1 to 3 heteroatoms which are preferably selected from nitrogen, oxygen and sulfur, or a bi- or tricyclic radical wherein, for example, one or two carbocyclic radicals, such as e.g. benzene radicals, are annellated (fused) to the mentioned monocyclic radical. If a heterocyclic radical contains a fused carbocyclic radical then the heterocyclic radical may also be attached to the rest of the molecule of formula I via a ring atom of the fused carbocyclic radical. The heterocyclic radical (including the fused carbocyclic radical(s) if present) is optionally substituted by one or more, preferably by one or two, radicals such as e.g. unsubstituted or substituted lower alkyl, amino, N-lower alkylamino, N,N-di-lower alkylamino, N-lower alkanoylamino, N,N-di-lower alkanoylamino, hydroxy, lower alkoxy, lower alkanoyl, lower alkanoyloxy, cyano, nitro, carboxy, lower alkoxycarbonyl, carbamoyl, N-lower alkyl-carbamoyl, N,N-di-lower alkyl-carbamoyl, amidino, guanidino, ureido, mercapto, lower alkylthio, or halogen.

Most preferably a heterocyclic radical is pyrrolidinyl, piperidyl, lower alkyl-piperazinyl, di-lower alkyl-piperazinyl, morpholinyl, tetrahydropyranyl, pyridyl, pyridyl substituted by hydroxy or lower alkoxy, or benzodioxolyl, especially pyrrolidinyl, piperidyl, lower alkyl-piperazinyl, di-lower alkyl-piperazinyl or morpholinyl.

A heterocyclic radical $R_1$ is as defined above for a heterocyclic radical with the proviso that it is bonded to Q via a ring carbon atom if X is not present. Preferably a heterocyclic radical $R_1$ is benzodioxolyl, pyridyl substituted by hydroxy or lower alkoxy, or especially preferred indolyl substituted by halogen and lower alkyl. If $R_1$ is pyridyl substituted by hydroxy then the hydroxy group is preferably attached to a ring carbon atom adjacent to the ring nitrogen atom.

An unsubstituted or substituted aromatic radical $R_1$ has up to 20 carbon atoms and is unsubstituted or substituted, for example in each case unsubstituted or substituted phenyl. Preferably an unsubstituted aromatic radical $R_1$ is phenyl. A substituted aromatic radical $R_1$ is preferably phenyl substituted by one or more substituents selected independently of one another from the group consisting of unsubstituted or substituted lower alkyl, amino, N-lower alkylamino, N,N-di-lower alkylamino, N-lower alkanoylamino, N,N-di-lower alkanoylamino, hydroxy, lower alkoxy, lower alkanoyl, lower alkanoyloxy, cyano, nitro, carboxy, lower alkoxycarbonyl, carbamoyl, N-lower alkyl-carbamoyl, N,N-di-lower alkyl-carbamoyl, amidino, guanidino, ureido, mercapto, lower alkylthio and halogen. Most preferably a substituted aromatic radical $R_1$ is phenyl substituted by one or more radicals selected independently of one another from the group consisting of lower alkyl, amino, hydroxy, lower alkoxy, halogen and benzyloxy.

Halogen is primarily fluoro, chloro, bromo or iodo, especially fluoro, chloro or bromo.

$C_1$-$C_7$-alkylene may be branched or unbranched and is in particular $C_1$-$C_3$-alkylene.

$C_1$-$C_7$-alkylene G is preferably $C_1$-$C_3$-alkylene, most preferably methylene (-$CH_2$-).

If G is not $C_1$-$C_7$-atkytene it preferably represents -C(=O)-.

$C_1$-$C_7$-alkylene X is preferably $C_1$-$C_3$-alkylene, most preferably methylene (-$CH_2$-) or ethan-1,1-diyl (-CH($CH_3$)-).

Q is preferably -NH-.

Salts are especially the pharmaceutically acceptable salts of compounds of formula I.

Such salts are formed, for example, as acid addition salts, preferably with organic or inorganic acids, from compounds of formula I with a basic nitrogen atom, especially the pharmaceutically acceptable salts.

In the presence of negatively charged radicals, such as carboxy or sulfo, salts may also be formed with bases, e.g. metal or ammonium salts, such as alkali metal or alkaline earth metal salts, or ammonium salts with ammonia or suitable organic amines, such as tertiary monoamines.

In the presence of a basic group and an acid group in the same molecule, a compound of formula I may also form internal salts.

For isolation or purification purposes it is also possible to use pharmaceutically unacceptable salts, for example picrates or perchlorates. Only the pharmaceutically acceptable salts or free compounds (if the occasion arises, in the form of pharmaceutical compositions) attain therapeutic use, and these are therefore preferred.

In view of the close relationship between the compounds in free form and in the form of their salts, including those salts that can be used as intermediates, for example in the purification or identification of the present compounds, hereinbefore and hereinafter any reference to the free compounds is to be understood as referring also to the corresponding salts, as appropriate and expedient.

The compounds of formula I have valuable, pharmacologically useful properties. In particular they exhibit specific inhibitory activities that are of pharmacological interest. They are effective especially as protein tyrosine kinase inhibitors and/or (furthermore) as inhibitors of serine/threonine protein kinases; they exhibit, for example, powerful inhibition of the tyrosine kinase activity of the epidermal growth factor receptor (EGF-R) and of ErbB-2 kinase. These two protein tyrosine kinase receptors, together with their family members ErbB-3 and ErbB-4, play a key role in signal transmission in a large number of mammalian cells, including human cells, especially epithelial cells, cells of the immune system and cells of the central and peripheral nervous system. For example, in various cell types, EGF-induced activation of receptor-associated protein tyrosine kinase is a prerequisite for cell division and hence for the proliferation of the cell population. Most importantly, overexpression of the EGF-R (HER-1) and/or ErbB-2 (HER-2) has been observed in substantial fractions of many human tumours. EGF-R, e.g., was found to be overexpressed in non small-cell lung cancers, squameous carcinoma (head and neck), breast, gastric, ovarian, colon and prostate cancers as well as in gliomas. ErbB-2 was found to be overexpressed in squameous carcinoma (head and neck), breast, gastric, and ovarian cancers as well as in gliomas.

In addition to inhibiting the tyrosine kinase activity of the EGF-R, the compounds of formula I also inhibit to varying extents other protein tyrosine kinases that are involved in signal transmission mediated by trophic factors, specially the vascular endothelial growth factor (VEGF) receptor family (e.g. KDR, Flt-1, Flt-3) but also abl kinase, especially v-abl, kinases from the family of Src, especially c-Src, Lck and Fyn, the other members of the EGF receptor family such as ErbB-3 (HER-3) and ErbB-4 (HER-4), CSF-1, Kit, FGF receptor and the cyclin-dependent kinases CDK1 and CDK2, all of which play a part in growth regulation and transformation in mammalian cells, including human cells.

The inhibition of EGF-R tyrosine kinase activity can be demonstrated using known methods, for example using the recombinant intracellular domain of the EGF-receptor [EGF-R ICD; see, for example, E. McGlynn et al., Europ. J. Biochem. 207, 265-275 (1992)]. Compared with the control without inhibitor, the compounds of formula I inhibit the enzyme activity by 50 % ($IC_{50}$), for example in a concentration of from 0.0005 to 0.5 μM, especially from 0.001 to 0.1 μM.

As well as or instead of inhibiting EGF-R tyrosine kinase activity, the compounds of formula I also inhibit other members of this family of receptors, like ErbB-2. The inhibitory activity ($IC_{50}$) is approximately in the range of 0.001 to 0.5 μM. The inhibition of ErbB-2 tyrosine kinase (HER-2) can be determined, for example, analogously to the method used for EGF-R protein tyrosine kinase [see C. House et al., Europ. J. Biochem. 140, 363-367 (1984)]. The ErbB-2 kinase can be isolated, and its activity determined, by means of protocols known *per se*, for example in accordance with T. Akiyama et al., Science 232, 1644 (1986).

Surprisingly, the compounds of formula I especially also inhibit the tyrosine kinase activity of the VEGF receptor family very potently. The compounds of the present invention are therefore very effective dual inhibitors of EGF- and VEGF-receptor family members. For inhibition of KDR and Flt-1 and inhibition of growth factor-induced proliferation of HUVECS see J. Wood et al., Cancer Res. 60, 2178-2189 (2000). The compounds of formula I inhibit e.g. the KDR tyrosine kinase activity with an $IC_{50}$ of from about 1 nM to about 1 μM. especially from about 5 nM to about 0.5 μM.

The action of the compounds of formula I on EGF-induced phosphorylation of the EGF-R can be determined in the human A431 epithelial carcinoma cell line by means of an ELISA which is described in U. Trinks et al., J. Med. Chem. 37:7, 1015-1027 (1994). In that test (EGF-R ELISA) the compounds of formula I exhibit an $IC_{50}$ of approximately from 0.001 to 1 μM.

The compounds of formula I potently inhibit the growth of EGF-R overexpressing NCI-H596 non-small cell lung carcinoma cells [see e.g. W. Lei, et al., Anticancer Res. 19(1A), 221-228 (1999)] at an $IC_{50}$ of approximately 0.01 to 1 μM. In the same range of activity, the compounds of formula I also potently inhibit the growth of ErbB-2-overexpressing BT474 human breast cancer cells. The test procedures are adapted from T. Meyer et al., Int. J. Cancer 43, 851 (1989). The inhibitory activity of the compounds of formula I is determined, briefly, as follows: NCI-H596 cells (10 000/microtitre plate well) are transferred to 96-well microtitre plates. The test compounds [dissolved in dimethyl sulfoxide (DMSO)] are added in a series of concentrations (dilution series) in such a manner that the final concentration of DMSO is not greater than 1 % (v/v). After the addition, the plates are incubated for three days during which the control cultures without test compound are able to undergo at least three cell-division cycles. The growth of the NCI-H596 cells is measured by means of methylene blue staining: after the incubation the cells are fixed with glutaraldehyde, washed with water and stained with 0.05 % methylene blue. After a washing step the stain is eluted with 3 % HCl and the optical density (OD) per well of the microtitre plate is measured using a Titertek Multiskan (Titertek, Huntsville, AL, USA) at 665 nm. $IC_{50}$ values are determined by a computer-aided system using the formula:

$$IC_{50} = [(OD_{test} - OD_{start})/(OD_{control} - OD_{start})] \times 100.$$

The $IC_{50}$ value in those experiments is given as that concentration of the test compound in question that results in a cell count that is 50 % lower than that obtained using the control without inhibitor. The compounds of formula I exhibit

inhibitory activity with an $IC_{50}$ in the range from approximately 0.01 to 1 μM.

The compounds of formula I exhibit inhibition of the growth of tumour cells also *in vivo*, as shown, for example, by the test described below: the test is based on inhibition of the growth of the human squamous lung carcinoma cell line NCI-H596 [ATCC HTB 178; American Type Culture Collection, Rockville, Maryland, USA; see Santon, J.B., et al., Cancer Research 46, 4701-4705 (1986) and Ozawa, S., et al., Int. J. Cancer 40, 706-710 (1987)], which is transplanted into female BALB/c nude mice (Bomholtgard, Denmark). That carcinoma exhibits a growth that correlates with the extent of the expression of the EGF-R. Tumours are established after subcutaneous (s.c.) injection of cells [a minimum of 2 x $10^6$ cells in 100 μl phosphate-buffered saline (PBS) or medium] in carrier mice (4-8 mice). Injections are made s.c. in the left flank of the mouse mid-way between the tail and the head. The resulting tumours are serially passaged for a minimum of three consecutive transplantations prior to start of the treatment. During this time tumour growth rates stabilize. Tumours are not passaged more than 12 times. For the therapy experiment tumour fragments of roughly 25 mg are transplanted s.c. into the left flank of the animals using a 13-gauge trocar needle under Forene® (Abbott, Schwitzerland) anesthesia. Tumour growth and body weights are monitored twice per week. All treatments are initiated when the tumour attains a volume of 100 to 250 $mm^3$. The tumour volumes are calculated using the known formula Length x $Diameter^2$ x π/6 [see Evans, B.D., et al., Brit. J. Cancer 45, 466-8 (1982)]. Antitumour activity is expressed as T/C % (mean increase of tumour volumes of treated animals divided by the mean increase of tumour volumes of control animals multiplied by 100 %). At a dose of from 3 to 100 mg/kg of active ingredient, distinct inhibition of the tumour growth is found, for example T/C % values of less than 50.

The compounds of formula I may inhibit other protein tyrosine kinases that are involved in signal transmission mediated by trophic factors, for example abl kinase, such as especially v-abl kinase ($IC_{50}$ for example from 0.01 to 5 μM), kinases from the family of the src kinases, such as especially c-src kinase ($IC_{50}$ for example from 0.1 to 10 μM) and serine/threonine kinases, for example protein kinase C, all of which are involved in growth regulation and transformation in mammalian cells, including human cells.

The above-mentioned inhibition of v-abl tyrosine kinase is determined by the methods of N. Lydon et al., Oncogene Research 5, 161-173 (1990) and J. F. Geissler et al., Cancer Research 52, 4492-4498 (1992). In those methods [$Val^5$]-angiotensin II and [$\gamma$-$^{32}P$]-ATP are used as substrates.

The compounds of formula I which inhibit the tyrosine kinase activity of the EGF-R or of the other protein tyrosine kinases mentioned are therefore useful, for example, in the treatment of benign or malignant tumours. The compounds of formula I are e.g. able to simultaneously inhibit the growth of tumors with deregulated EGF-R and/or ErbB-2 activity as well as to inhibit the vascularisation of solid tumors triggered by VEGF. This combined activity leads to an improved antitumour effect (see also WO 02/41882). Moreover, the use of a dual inhibitor reduces the risk of drug-drug interactions and further reduces the total drug load as compared to a combination therapy. The compounds of formula I are capable of slowing down tumor growth or effecting tumour regression and of preventing the formation of tumour metastases and the growth of micrometastases. They can be used especially in the case of epidermal hyperproliferation (psoriasis), in the treatment of neoplasias of epithelial character, e.g. mammary carcinomas, and in leukaemias. In addition, the compounds of formula I can be used in the treatment of those disorders of the immune system in which several or, especially, individual protein tyrosine kinases and/or (furthermore) serine/threonine protein kinases are involved; the compounds of formula I can also be used in the treatment of those disorders of the central or peripheral nervous system in which signal transmission by several or, especially, a single protein tyrosine kinase(s) and/or (furthermore) serine/threonine protein kinase(s) is/are involved.

In general, the present invention relates also to the use of the compounds of formula I for the inhibition of the mentioned protein kinases, in particular to their use for the dual inhibition of EGF- and VEGF-receptor family members.

The compounds according to the invention can be used both alone and in combination with other pharmacologically active compounds, for example together with inhibitors of the enzymes of polyamine synthesis, inhibitors of protein kinase C, inhibitors of other tyrosine kinases, cytokines, negative growth regulators, for example TGF-β or IFN-β, aromatase inhibitors, antioestrogens and/or cytostatic drugs.

With the groups of compounds of formula according to the invention mentioned herein, definitions of substituents from the general definitions mentioned hereinbefore may reasonably be used, for example, to replace more general definitions with more specific definitions or especially with definitions characterized as being preferred.

Further disclosed herein is a compound of formula I, wherein

$R_1$ is a heterocyclic radical or an unsubstituted or substituted aromatic radical;
G is $C_1$-$C_7$-alkylene;
Q is -NH- or -O-; and
X is either not present or $C_1$-$C_7$-alkylene, with the proviso that a heterocyclic radical $R_1$ is bonded via a ring carbon atom if X is not present;

or a salt thereof.

Further disclosed herein is a compound of formula I, wherein

$R_1$ is a heterocyclic radical or an unsubstituted or substituted aromatic radical;
G is $C_1$-$C_7$-alkylene;
Q is -NH-; and
X is either not present or $C_1$-$C_7$-alkylene, with the proviso that a heterocyclic radical $R_1$ is bonded via a ring carbon atom if X is not present;

or a salt thereof.

Further disclosed herein is a compound of formula I, wherein

$R_1$ is a heterocyclic radical containing up to 20 carbon atoms or an unsubstituted or substituted aromatic radical having up to 20 carbon atoms;
G is $C_1$-$C_3$-alkylene;
Q is -NH-; and
X is either not present or $C_1$-$C_3$-alkylene, with the proviso that a heterocyclic radical $R_1$ is bonded via a ring carbon atom if X is not present;

or a salt thereof.

Special preference is given to a compound of formula I, wherein

$R_1$ is phenyl, benzodioxolyl, pyridyl substituted by hydroxy or lower alkoxy having up to and including a maximum of 7 carbon atoms, or phenyl substituted by one or more radicals selected independently of one another from the group consisting of lower alkyl having up to and including a maximum of 7 carbon atoms, hydroxy, lower alkoxy having up to and including a maximum of 7 carbon atoms, halogen and benzyloxy;
G is $-CH_2-$;
Q is -NH-; and
X is either not present, $-CH_2-$ or $-CH(CH_3)-$, with the proviso that substituted pyridyl $R_1$ is bonded via a ring carbon atom if X is not present;

or a salt thereof.

Special preference is also given to a compound of formula I wherein $C_1$-$C_7$-alkylene G is attached to the phenyl ring at position 3 or 4, most especially at position 4.

Most special preference is further given to a compound of formula I mentioned in the Examples below, or a salt, specially a pharmaceutically acceptable salt, thereof.

Also especially preferred are compounds of formula I, which according to the above-described tyrosine kinase inhibition assays - inhibit HER-1, HER-2 and KDR with $IC_{50}$ values of less than 300 nM, most preferably of less than 100 nM.

Very special preference is further given to compounds of formula I which inhibit the tyrosine kinase activity of at least one member of the EGF receptor family together with at least one member of the VEGF receptor family (dual inhibition of EGF- and VEGF-receptor family members) with $IC_{50}$ values in the range of 0.5 nM to 0.5 μM, especially in the range of 1 nM to 300 nM, based on the above-described tyrosine kinase inhibition assays.

Especially preferred are further also compounds of formula I in which G is $C_1$-$C_7$-alkylene since the amine group of such compounds allows to generate pharmaceutically acceptable salts of these compounds which in general leads to an increased solubility and to improved physico-chemical properties.

In a preferred embodiment, the invention relates to isolated compounds of formula I, especially to the isolated compound ((*R*)-1-phenyl-ethyl)-[6-(4-piperazin-1-ylmethyl-phenyl)-7*H*-pyrrolo[2,3-*d*]pyrimidin-4-yl]-amine, or salts, especially pharmaceutically acceptable salts, thereof.

The term "isolated" compound means that the compound is removed from its original environment (e.g., the natural environment if it is naturally occurring including the human or animal body that for example produces such a compound upon administration of another compound). For example, a naturally occurring compound in its natural environment is not isolated, but the same compound, separated from some or all of the co-existing materials in the natural system, is isolated, even if subsequently reintroduced into the natural system. Such compounds could be part of a composition and still be isolated, in that such composition is not part of its natural environment. Thus, the compound is at least partially purified relative to the natural environment in which it is found and is not, therefore, a product of nature.

In an important embodiment, the isolated compound is of sufficient purity to permit its use for pharmaceutical applications, particularly for the preparation of a pharmaceutical composition.

The compounds of formula I or salts thereof can be prepared according to known processes (see e.g. WO

03/013541 A1 published on 20 February 2003), especially whereby

a) in order to prepare a compound of formula I, wherein G is $C_1$-$C_7$-alkylene, a compound of the formula II

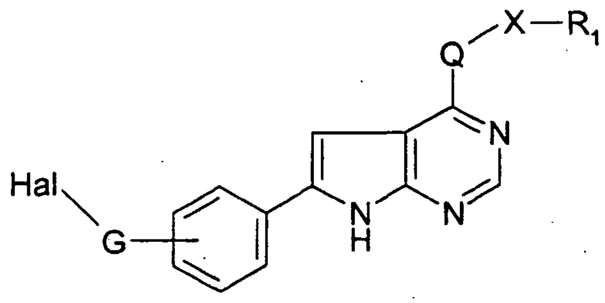

(II),

wherein Hal is halogen, G is $C_1$-$C_7$-alkylene and $R_1$, Q and X have the meanings as defined for a compound of formula I, is reacted with piperazine;

b) in order to prepare a compound of formula I, wherein G is -C(=O)- or $C_1$-$C_6$-alkylene- C (=O)- wherein the carbonyl group is attached to the piperazine moiety, a compound of formula III

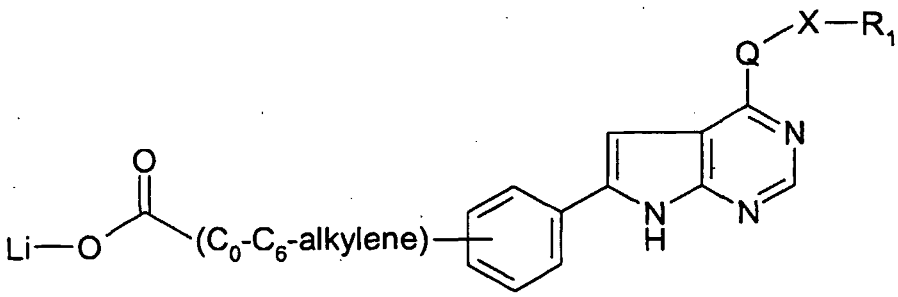

(III),

wherein the substituents and symbols have the meanings as defined for a compound of formula I, is reacted with piperazine; or

c) in order to prepare a compound of formula I, wherein G is $C_1$-$C_7$-alkylene, a compound of formula I, wherein G is -C(=O)- or $C_1$-$C_6$-alkylene-C(=O)- wherein the carbonyl group is attached to the piperazine moiety, is reacted with a reducing agent to produce the corresponding compound in which G is $C_1$-$C_7$-alkylene;

whereby functional groups which are present in the starting compounds of processes a) to c) and are not intended to take part in the reaction, are present in protected form if necessary, and protecting groups that are present are cleaved, whereby the said starting compounds may also exist in the form of salts provided that a salt-forming group is present and a reaction in salt form is possible;
and, if so desired, a compound of formula I thus obtained is converted into another compound of formula I, a free compound of formula I is converted into a salt, an obtained salt of a compound of formula I is converted into the free compound or another salt; and/or a mixture of isomeric compounds of formula I is separated into the individual isomers.

<u>Description of the process variants</u>:

Regarding process a):

The reaction between a compound of formula II and piperazine (preferably protected at one of its nitrogen ring atoms, such as especially *N-tert*-butoxycarbonyl-piperazine) preferably takes place in a suitable inert solvent, especially *N,N*-dimethylformamide, in the presence of a base such as potassium carbonate, at temperatures from room temperature

(RT) to 100 °C. Alternatively, the reaction between a compound of formula II and piperazine (preferably in protected form, such as *N-tert*-butoxycarbonyl-piperazine) takes place in a suitable solvent, e.g. lower alcohols, such as ethanol, in the presence of for example a suitable catalyst such as NaI, preferably at the reflux temperature of the solvent employed. In a compound of formula II, Hal is preferably chloro.

Regarding process b):

The reaction of a compound of formula III with piperazine (preferably protected at one of its nitrogen ring atoms, such as especially *N-tert*-butoxycarbonyl-piperazine) preferably takes place in a suitable inert solvent such as *N*, *N*-dimethylformamide and in an inert, for example an argon or nitrogen, atmosphere, in the presence of diethyl-cyanophosphonate, preferably at about 0 °C.

Regarding process c):

The reducing agent used in process c) is preferably lithium aluminium hydride or diisobutyl-aluminium hydride. The reaction preferably takes place under those conditions described in Example 79 or 141 of WO 03/013541 A1, respectively.

Additional process steps

In the additional process steps, carried out as desired, functional groups of the starting compounds which should not take part in the reaction may be present in unprotected form or may be protected for example by one or more protecting groups. The protecting groups are then wholly or partly removed according to one of the known methods, especially according to the methods described in the Examples.
Protecting groups, and the manner in which they are introduced and removed are described, for example, in "Protective Groups in Organic Chemistry", Plenum Press, London, New York 1973, and in "Methoden der organischen Chemie", Houben-Weyl, 4th edition, Vol. 15/1, Georg-Thieme-Verlag, Stuttgart 1974 and in Theodora W. Greene, "Protective Groups in Organic Synthesis", John Wiley & Sons, New York 1981. A characteristic of protecting groups is that they can be removed readily, i.e. without the occurrence of undesired secondary reactions, for example by solvolysis, reduction, photolysis or alternatively under physiological conditions.

The end products of formula I may, however, also contain substituents that can be used as protecting groups in starting materials for the preparation of other end products of formula I. Thus, within the scope of this text, only a readily removable group that is not a constituent of the particular desired end product of formula I is designated a "protecting group", unless the context indicates otherwise.

General process conditions

All process steps described here can be carried out under known reaction conditions, preferably under those specifically mentioned, in the absence of or usually in the presence of solvents or diluents, preferably those that are inert to the reagents used and able to dissolve them, in the absence or presence of catalysts, condensing agents or neutralising agents, for example ion exchangers, typically cation exchangers, for example in the $H^+$ form, depending on the type of reaction and/or reactants at reduced, normal, or elevated temperature, for example in the range from -100 °C to about 190 °C, preferably from about
-80 °C to about 150 °C, for example at -80 to -60 °C, at RT, at -20 to 40 °C, at 0 to 100 °C or at the boiling point of the solvent used, under atmospheric pressure or in a closed vessel, if need be under pressure, and/or in an inert, for example an argon or nitrogen, atmosphere.

The invention relates also to those embodiments of the process in which one starts from a compound obtainable at any stage as an intermediate and carries out the missing steps, or breaks off the process at any stage, or forms a starting material under the reaction conditions, or uses said starting material in the form of a reactive derivative or salt, or produces a compound obtainable by means of the process according to the invention under those process conditions, and further processes the said compound *in situ*. In the preferred embodiment, one starts from those starting materials which lead to the compounds described hereinabove as preferred.

In the preferred embodiment, a compound of formula I is prepared according to the processes and process steps defined in the Examples.

The compounds of formula I, including their salts, are also obtainable in the form of hydrates, or their crystals can include for example the solvent used for crystallisation (present as solvates).

Starting materials

New starting materials and/or intermediates, as well as processes for the preparation thereof, are likewise the subject of this invention. In the preferred embodiment, such starting materials are used and reaction conditions so selected as to enable the preferred compounds to be obtained.

The starting materials used in the above described processes a) to c) are known, capable of being prepared according to known processes (see also EP 682 027, WO 97/02266, WO 97/27199, WO 98/07726 and WO 03/013541 A1), or commercially obtainable; in particular, they can be prepared using processes as described in the Examples.

In the preparation of starting materials, existing functional groups which do not participate in the reaction should, if necessary, be protected. Preferred protecting groups, their introduction and their removal are described above or in the Examples. In place of the respective starting materials and transients, salts thereof may also be used for the reaction, provided that salt-forming groups are present and the reaction with a salt is also possible. Where the term starting materials is used hereinbefore and hereinafter, the salts thereof are always included, insofar as reasonable and possible.

A compound of formula II can be prepared for example by reacting a compound of formula IV

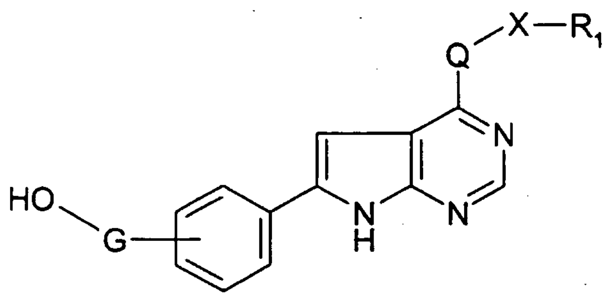

(IV),

wherein G is $C_1$-$C_7$-alkylene and $R_1$, Q and X have the meanings as defined for a compound of formula I, with e.g. thionyl halogenide, preferably thionyl choride, in the presence or absence of pyridine, in an inert solvent, for example toluene or in a 1:1 mixture of acetonitrile and dioxane, preferably at -10 to 0 °C or at RT.

A compound of formula IV can be prepared for example by reacting a compound of formula V

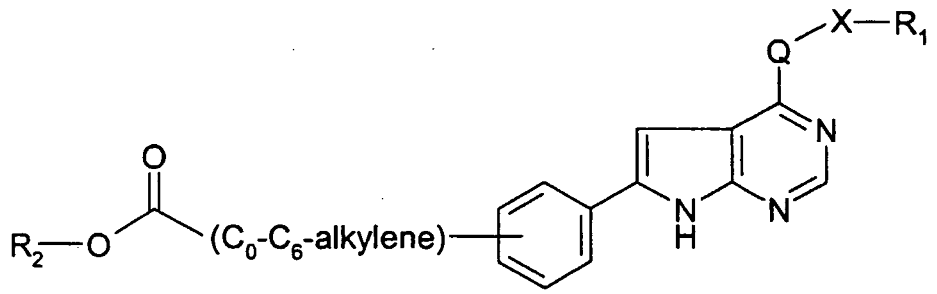

(V),

wherein $R_2$ is lower alkyl, especially methyl or ethyl, and $R_1$, Q and X have the meanings as defined for a compound of formula I, with lithium aluminium hydride, in an inert solvent, especially ethers, e.g. cyclic ethers such as tetrahydrofuran, preferably at the reflux temperature of the solvent employed. Alternatively, a compound of formula IV may be prepared by reacting a compound of formula V with diisobutyl-aluminium hydride, in an inert solvent, for example in tetrahydrofuran or in a 1:1 mixture of dichloromethane and dioxane, preferably at RT.

A compound of formula V wherein Q is -NH- can be prepared for example by reacting a compound of formula VI

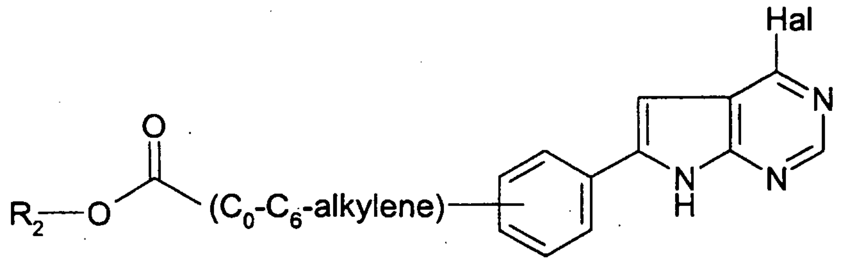

(VI),

wherein Hal is halogen, preferably chloro, and $R_2$ is as defined above for a compound of formula V, with a compound of the formula $H_2N$-X-$R_1$, wherein $R_1$ and X have the meanings as defined for a compound of formula I, (i) in a suitable solvent such as alcohols, especially lower alcohols such as n-butanol, preferably at the boiling temperature of the solvent employed or (ii) under catalytic conditions e.g. according to the Buchwald reaction conditions such as those described in Step 133.1 of Example 133 of WO 03/013541 A1.

A compound of formula V wherein Q is -O- can be prepared for example by reacting a compound of formula VI, which is preferably N-protected in the pyrrolo-pyrimidine moiety, with a compound of the formula HO-X-$R_1$, wherein $R_1$ and X have the meanings as defined for a compound of formula I, in a suitable inert solvent such as *N,N*-dimethylformamide and in the presence of a base such as potassium carbonate, at elevated temperatures, preferably at around 100 °C.

Alternatively, the carboxylic acid ester of a compound of formula VI may first be reduced to the corresponding alcohol, e.g. under conditions described above for the preparation of a compound of formula IV, and then either be reacted with a compound of the formula $H_2N$-X-$R_1$, e.g. under conditions described above for the preparation of a compound of formula V wherein Q is -NH-, or be reacted with a compound of the formula HO-X-$R_1$, e.g. under conditions described above for the preparation of a compound of formula V wherein Q is -O-.

A compound of formula III can be prepared for example by reacting a compound of formula V with LiOH, preferably in a mixture of dioxane and water, at elevated temperatures, preferably under those conditions described in Step 141.4 of Example 141 of WO 03/013541 A1.

The remaining starting materials are known, capable of being prepared according to known processes, or commercially available; or in particular, they can be prepared using processes as described in the Examples.

Pharmaceutical compositions, methods, and uses

The present invention relates also to pharmaceutical compositions that comprise a compound of formula I, or a pharmaceutically acceptable salt thereof, as active ingredient and that can be used especially in the treatment of the diseases mentioned at the beginning. Compositions for enteral administration, such as nasal, buccal, rectal or, especially, oral administration, and for parenteral administration, such as intravenous, intramuscular or subcutaneous administration, to warm-blooded animals, especially humans, are especially preferred. The compositions contain the active ingredient alone or, preferably, together with a pharmaceutically acceptable carrier. The dosage of the active ingredient depends upon the disease to be treated and upon the species, its age, weight, and individual condition, the individual pharmacokinetic data, and the mode of administration.

The present invention also relates to pro-drugs of a compound of formula I that convert *in vivo* to the compound of formula I as such. Any reference to a compound of formula I is therefore to be understood as referring also to the corresponding pro-drugs of the compound of formula I, as appropriate and expedient.

The invention relates also to compounds of formula I, or a pharmaceutically acceptable salt thereof, as such or in the form of a pharmaceutical composition, for use in a method for the prophylactic or especially therapeutic treatment of the human or animal body, to a process for the preparation thereof (especially in the form of compositions for the treatment of tumours) and to a method of treating proliferative diseases, primarily tumour diseases, especially those mentioned above.

The invention relates also to processes and to the use of compounds of formula I, or a pharmaceutically acceptable salt thereof, for the preparation of pharmaceutical compositions which comprise compounds of formula I, or a pharmaceutically acceptable salt thereof, as active component (active ingredient).

If desired, the said pharmaceutical compositions may also contain further active components, for example cytostatics, and/or may be used in combination with known therapeutic processes, for example the administration of hormones or radiation.

Preference is given for a pharmaceutical composition which is suitable for administration to a warm-blooded

animal, especially humans or commercially useful mammals suffering from a disease which responds to an inhibition of a protein tyrosine kinase, especially to a dual inhibition of EGF- and VEGF-receptor family members, especially a neoplastic disease, comprising an effective quantity of a compound of formula I for the inhibition of a protein tyrosine kinase, especially for the dual inhibition of EGF- and VEGF-receptor family members, or a pharmaceutically acceptable salt thereof, together with at least one pharmaceutically acceptable carrier.

A pharmaceutical composition for the prophylactic or especially therapeutic management of neoplastic and other proliferative diseases of a warm-blooded animal, especially a human or a commercially useful mammal requiring such treatment, especially suffering from such a disease, comprising as active ingredient in a quantity that is prophylactically or especially therapeutically active against said diseases a compound of formula I, or a pharmaceutically acceptable salt thereof, is likewise preferred.

The pharmaceutical compositions comprise from approximately 1 % to approximately 95% active ingredient, single-dose administration forms comprising in the preferred embodiment from approximately 20% to approximately 90% active ingredient and forms that are not of single-dose type comprising in the preferred embodiment from approximately 5% to approximately 20% active ingredient. Unit dose forms are, for example, coated and uncoated tablets, ampoules, vials, suppositories or capsules. Examples are capsules containing from about 0.05 g to about 1.0 g of active substance.

The pharmaceutical compositions of the present invention are prepared in a manner known per se, for example by means of conventional mixing, granulating, coating, dissolving or lyophilising processes.

The invention relates likewise to a process or a method for the treatment of one of the pathological conditions mentioned hereinabove, especially a disease which responds to an inhibition of a protein tyrosine kinase, especially to a dual inhibition of EGF- and VEGF-receptor family members, especially a corresponding neoplastic disease. The compounds of formula I, or pharmaceutically acceptable salts thereof, can be administered as such or in the form of pharmaceutical compositions, prophylactically or therapeutically, preferably in an amount effective against the said diseases, to a warm-blooded animal, for example a human, requiring such treatment, the compounds especially being used in the form of pharmaceutical compositions. In the case of an individual having a bodyweight of about 70 kg the daily dose administered is from approximately 0.1 g to approximately 5 g, preferably from approximately 0.5 g to approximately 2 g, of a compound of the present invention.

The present invention relates especially also to the use of a compound of formula I, or a pharmaceutically acceptable salt thereof, especially a compound of formula I which is said to be preferred, or a pharmaceutically acceptable salt thereof, as such or in the form of a pharmaceutical composition with at least one pharmaceutically acceptable carrier, for the therapeutic and also prophylactic management of one or more of the diseases mentioned hereinabove, preferably a disease which responds to an inhibition of a protein tyrosine kinase, especially to a dual inhibition of EGF- and VEGF-receptor family members, especially a neoplastic disease, in particular if the said disease responds to an inhibition of a protein tyrosine kinase, especially to a dual inhibition of EGF- and VEGF-receptor family members.

The present invention relates especially also to the use of a compound of formula I, or a pharmaceutically acceptable salt thereof, especially a compound of formula I which is said to be preferred, or a pharmaceutically acceptable salt thereof, for the preparation of a pharmaceutical composition for the therapeutic and also prophylactic management of one or more of the diseases mentioned hereinabove, especially a neoplastic disease, in particular if the disease responds to an inhibition of a protein tyrosine kinase, especially to a dual inhibition of EGF- and VEGF-receptor family members.

A compound of formula I may also be used to advantage in combination with other antiproliferative agents. Such antiproliferative agents include, but are not limited to aromatase inhibitors, antiestrogens, topoisomerase I inhibitors, topoisomerase II inhibitors, microtubule active agents, alkylating agents, histone deacetylase inhibitors, famesyl transferase inhibitors, COX-2 inhibitors, MMP inhibitors, mTOR inhibitors, antineoplastic antimetabolites, platin compounds, compounds decreasing the protein kinase activity and further anti-angiogenic compounds, gonadorelin agonists, antiandrogens, bengamides, bisphosphonates, antiproliferative antibodies and temozolomide (TEMODAL®).

The term "aromatase inhibitors" as used herein relates to compounds which inhibit the estrogen production, i.e. the conversion of the substrates androstenedione and testosterone to estrone and estradiol, respectively. The term includes, but is not limited to steroids, especially exemestane and formestane and, in particular, non-steroids, especially aminoglutethimide, vorozole, fadrozole, anastrozole and, very especially, letrozole. Exemestane can be administered, e.g., in the form as it is marketed, e.g. under the trademark AROMASIN™. Formestane can be administered, e.g., in the form as it is marketed, e.g. under the trademark LENTARON™. Fadrozole can be administered, e.g., in the form as it is marketed, e.g. under the trademark AFEMA™. Anastrozole can be administered, e.g., in the form as it is marketed, e.g. under the trademark ARIMIDEX™. Letrozole can be administered, e.g., in the form as it is marketed, e.g. under the trademark FEMARA™ or FEMAR™. Aminoglutethimide can be administered, e.g., in the form as it is marketed, e.g. under the trademark ORIMETEN™.

A combination of the invention comprising an antineoplastic agent which is an aromatase inhibitor is particularly useful for the treatment of hormone receptor positive breast tumors.

The term "antiestrogens" as used herein relates to compounds which antagonize the effect of estrogens at the estrogen receptor level. The term includes, but is not limited to tamoxifen, fulvestrant, raloxifene and raloxifene hydrochloride. Tamoxifen can be administered, e.g., in the form as it is marketed, e.g. under the trademark NOLVADEX™. Raloxifene hydrochloride can be administered, e.g., in the form as it is marketed, e.g. under the trademark EVISTA™. Fulvestrant can be formulated as disclosed in US 4,659,516 or it can be administered, e.g., in the form as it is marketed, e.g. under the trademark FASLODEX™.

The term "topoisomerase I inhibitors" as used herein includes, but is not limited to topotecan, irinotecan, 9-nitrocamptothecin and the macromolecular camptothecin conjugate PNU-166148 (compound A1 in WO99/17804). Irinotecan can be administered, e.g., in the form as it is marketed, e.g. under the trademark CAMPTOSAR™. Topotecan can be administered, e.g., in the form as it is marketed, e.g. under the trademark HYCAMTIN™.

The term "topoisomerase II inhibitors" as used herein includes, but is not limited to the antracyclines doxorubicin (including liposomal formulation, e.g. CAELYX™), epirubicin, idarubicin and nemorubicin, the anthraquinones mitoxantrone and losoxantrone, and the podophillotoxines etoposide and teniposide. Etoposide can be administered, e.g., in the form as it is marketed, e.g. under the trademark ETOPOPHOS™. Teniposide can be administered, e.g., in the form as it is marketed, e.g. under the trademark VM 26-BRISTOL ™. Doxorubicin can be administered, e.g., in the form as it is marketed, e.g. under the trademark ADRIBLASTIN™. Epirubicin can be administered, e.g., in the form as it is marketed, e.g. under the trademark FARMORUBICIN™. Idarubicin can be administered, e.g., in the form as it is marketed, e.g. under the trademark ZAVEDOS™. Mitoxantrone can be administered, e.g., in the form as it is marketed, e.g. under the trademark NOVANTRON™.

The term "microtubule active agents" relates to microtubule stabilizing and microtubule destabilizing agents including, but not limited to the taxanes paclitaxel and docetaxel, the vinca alkaloids, e.g., vinblastine, especially vinblastine sulfate, vincristine especially vincristine sulfate, and vinorelbine, discodermolide and epothilones, such as epothilone B and D. Docetaxel can be administered, e.g., in the form as it is marketed, e.g. under the trademark TAXOTERE™. Vinblastine sulfate can be administered, e.g., in the form as it is marketed, e.g. under the trademark VINBLASTIN R.P.™. Vincristine sulfate can be administered, e.g., in the form as it is marketed, e.g. under the trademark FARMISTIN™. Discodermolide can be obtained, e.g., as disclosed in US 5,010,099.

The term "alkylating agents" as used herein includes, but is not limited to cyclophosphamide, ifosfamide and melphalan. Cyclophosphamide can be administered, e.g., in the form as it is marketed, e.g. under the trademark CYCLOSTIN™. Ifosfamide can be administered, e.g., in the form as it is marketed, e.g. under the trademark HOLOXAN™.

The term "histone deacetylase inhibitors" relates to compounds which inhibit the histone deacetylase and which possess antiproliferative activity.

The term "farnesyl transferase inhibitors" relates to compounds which inhibit the farnesyl transferase and which possess antiproliferative activity.

The term "COX-2 inhibitors" relates to compounds which inhibit the cyclooxygenase type 2 enyzme (COX-2) and which possess antiproliferative activity such as celecoxib (Celebrex®), rofecoxib (Vioxx®) and lumiracoxib (COX189).

The term "MMP inhibitors" relates to compounds which inhibit the matrix metalloproteinase (MMP) and which possess antiproliferative activity.

The term "mTOR inhibitors" relates to compounds which inhibit the mammalian target of rapamycin (mTOR) and which possess antiproliferative activity such as sirolimus (Rapamune®), everolimus (Certican™), CCI-779 and ABT578.

The term "antineoplastic antimetabolites" includes, but is not limited to 5-fluorouracil, tegafur, capecitabine, cladribine, cytarabine, fludarabine phosphate, fluorouridine, gemcitabine, 6-mercaptopurine, hydroxyurea, methotrexate, edatrexate and salts of such compounds, and furthermore ZD 1694 (RALTITREXED™), LY231514 (ALIMTA™), LY264618 (LOMOTREXOL™) and OGT719.

The term "platin compounds" as used herein includes, but is not limited to carboplatin, cisplatin and oxaliplatin. Carboplatin can be administered, e.g., in the form as it is marketed, e.g. under the trademark CARBOPLAT™. Oxaliplatin can be administered, e.g., in the form as it is marketed, e.g. under the trademark ELOXATIN™.

The term "compounds decreasing the protein kinase activity and further anti-angiogenic compounds" as used herein includes, but is not limited to compounds which decrease the activity of e.g. the Vascular Endothelial Growth Factor (VEGF), the Epidermal Growth Factor (EGF), c-Src, protein kinase C, Platelet-derived Growth Factor (PDGF), Bcr-Abl tyrosine kinase, c-kit, Flt-3 and Insulin-like Growth Factor I Receptor (IGF-IR) and Cyclin-dependent kinases (CDKs), and anti-angiogenic compounds having another mechanism of action than decreasing the protein kinase activity. Compounds which decrease the activity of VEGF are especially compounds which inhibit the VEGF receptor, especially the tyrosine kinase activity of the VEGF receptor, and compounds binding to VEGF, and are in particular those compounds, proteins and monoclonal antibodies generically and specifically disclosed in WO 98/35958 (describing compounds of formula I), WO 00/09495, WO 00/27820, WO 00/59509, WO 98/11223, WO 00/27819, WO 01/55114, WO 01/58899 and EP 0 769 947; those as described by M. Prewett et al in Cancer Research 59 (1999) 5209-5218, by F. Yuan et al in Proc. Natl. Acad. Sci. USA, vol. 93, pp. 14765-14770, December 1996, by Z. Zhu et al in Cancer Res. 58, 1998,

3209-3214, and by J. Mordenti et al in Toxicologic Pathology, vol. 27, no. 1, pp 14-21, 1999; in WO 00/37502 and WO 94/10202; Angiostatin™, described by M. S. O'Reilly et al, Cell 79, 1994, 315-328; and Endostatin™, described by M. S. O'Reilly et al, Cell 88, 1997, 277-285; compounds which decrease the activity of EGF are especially compounds which inhibit the EGF receptor, especially the tyrosine kinase activity of the EGF receptor, and compounds binding to EGF, and are in particular those compounds generically and specifically disclosed in WO 97/02266 (describing compounds of formula IV), EP 0 564 409, WO 99/03854, EP 0520722, EP 0 566 226, EP 0 787 722, EP 0 837 063, WO 98/10767, WO 97/30034,WO 97/49688, WO 97/38983 and, especially, WO 96/33980;
compounds which decrease the activity of c-Src include, but are not limited to, compounds inhibiting the c-Src protein tyrosine kinase activity as defined below and to SH2 interaction inhibitors such as those disclosed in WO97/07131 and WO97/08193;
compounds inhibiting the c-Src protein tyrosine kinase activity include, but are not limited to, compounds belonging to the structure classes of pyrrolopyrimidines, especially pyrrolo[2,3-d]pyrimidines, purines, pyrazopyrimidines, especially pyrazo[3,4-d]pyrimidines, pyrazopyrimidines, especially pyrazo[3,4-d]pyrimidines and pyridopyrimidines, especially pyrido[2,3-d]pyrimidines. Preferably, the term relates to those compounds disclosed in WO 96/10028, WO 97/28161, WO97/32879 and WO97/49706;
compounds which decreases the activity of the protein kinase C are especially those staurosporine derivatives disclosed in EP 0 296 110 (pharmaceutical preparation described in WO 00/48571) which compounds are protein kinase C inhibitors; further specific compounds that decrease protein kinase activity and which may also be used in combination with the compounds of the present invention are Imatinib (Gleevec®/Glivec®), PKC412, Iressa™ (ZD1839), PKI166, PTK787, ZD6474, GW2016, CHIR-200131, CEP-7055/CEP-5214, CP-547632 and KRN-633;
anti-angiogenic compounds having another mechanism of action than decreasing the protein kinase activity include, but are not limited to e.g. thalidomide (THALOMID), celecoxib (Celebrex), SU5416 and ZD6126.

The term "gonadorelin agonist" as used herein includes, but is not limited to abarelix, goserelin and goserelin acetate. Goserelin is disclosed in US 4,100,274 and can be administered, e.g., in the form as it is marketed, e.g. under the trademark ZOLADEX™. Abarelix can be formulated, eg. as disclosed in US 5,843,901.

The term "anti-androgens" as used herein includes, but is not limited to bicalutamide (CASODEX™), which can be formulated, e.g. as disclosed in US 4,636,505.

The term "bengamides" relates to bengamides and derivatives thereof having aniproliferative properties.

The term "bisphosphonates" as used herein includes, but is not limited to etridonic acid, clodronic acid, tiludronic acid, pamidronic acid, alendronic acid, ibandronic acid, risedronic acid and zoledronic acid. "Etridonic acid" can be administered, e.g., in the form as it is marketed, e.g. under the trademark DIDRONEL™. "Clodronic acid" can be administered, e.g., in the form as it is marketed, e.g. under the trademark BONEFOS™. "Tiludronic acid" can be administered, e.g., in the form as it is marketed, e.g. under the trademark SKELID™. "Pamidronic acid" can be administered, e.g., in the form as it is marketed, e.g. under the trademark AREDIA™. "Alendronic acid" can be administered, e.g., in the form as it is marketed, e.g. under the trademark FOSAMAX™. "Ibandronic acid" can be administered, e.g., in the form as it is marketed, e.g. under the trademark BONDRANAT™. "Risedronic acid" can be administered, e.g., in the form as it is marketed, e.g. under the trademark ACTONEL™. "Zoledronic acid" can be administered, e.g., in the form as it is marketed, e.g. under the trademark ZOMETA™.

The term "antiproliferative antibodies" as used herein includes, but is not limited to trastuzumab (Herceptin™), Trastuzumab-DM1, erlotinib (Tarceva™), bevacizumab (Avastin ™), rituximab (Rituxan®), PRO64553 (anti-CD40) and 2C4 Antibody.

For the treatment of AML, compounds of formula I can be used in combination with standard leukemia therapies, especially in combination with therapies used for the treatment of AML. In particular, compounds of formula I can be administered in combination with e.g. farnesyltransferase inhibitors and/or other drugs used for the treatment of AML, such as Daunorubicin, Adriamycin, Ara-C, VP-16, Teniposide, Mitoxantrone, Idarubicin, Carboplatinum and PKC412.

The structure of the active agents identified by code nos., generic or trade names may be taken from the actual edition of the standard compendium "The Merck Index" or from databases, e.g. Patents International (e.g. IMS World Publications).

The above-mentioned compounds, which can be used in combination with a compound of formula I, can be prepared and administered as described in the art such as in the documents cited above.

<u>Examples</u>:

The following Examples serve to illustrate the invention without limiting its scope.

Temperatures are measured in degrees Celsius. Unless otherwise indicated, the reactions take place at RT.

The $R_f$ values which indicate the ratio of the distance moved by each substance to the distance moved by the eluent front are determined on silica gel thin-layer plates (Merck, Darmstadt, Germany) by thin-layer chromatography using the respective named solvent systems.

HPLC conditions:

Column: (250 x 4.6 mm) packed with reversed-phase material C18-Nucleosil (5 μm mean particle size, with silica gel covalently derivatized with octadecylsilanes, Macherey & Nagel, Düren, Germany). Detection by UV absorption at 215 nm. The retention times ($t_R$) are given in minutes. Flow rate: 1 ml/min.

Gradient: 20% → 100% a) in b) for 14 min + 5 min 100% a). a): Acetonitrile + 0.05% trifluoroacetic acid (TFA); b): water + 0.05% TFA.

The short forms and abbreviations used have the following definitions:

| | |
|---|---|
| BOC | *tert*-butoxy-carbonyl |
| conc. | concentrated |
| DMF | N,N-dimethylformamide |
| MS-ES | mass spectroscopy (electron spray) |
| m.p. | melting point |
| TLC | thin-layer chromatography |
| $t_R$ | retention times |

Starting material: 4-{4-[4-((*R*)-1-Phenyl-ethylamino)-7*H*-pyrrolo[2,3-*d*]pyrimidin-6-yl]-benzyl}-piperazine-1-carboxylic acid *tert*-butyl ester

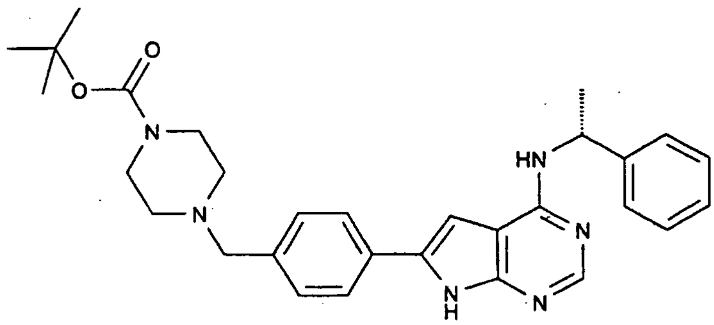

A mixture of 1.6 g (4 mmol) [6-(4-chloromethyl-phenyl)-7*H*-pyrrolo[2,3-*d*]pyrimidin-4-yl]-((*R*)-1-phenyl-ethyl)-amine (for the preparation see Example 9, Step 9.3 of WO 03/013541 A1) in 50 ml DMF is treated with 1.56 g (8.4 mmol) *N*-BOC-piperazine and 2.76 g (20 mmol) anhydrous potassium carbonate and the mixture heated to 65 °C for 1 hour. The reaction mixture is cooled and the inorganic salts removed by filtration (Hyflo Super Cel®; Fluka, Buchs, Switzerland). The DMF is evaporated under reduced pressure and the residue purified through flash chromatography using first dichloromethane/ethanol 95:5 and then dichloromethane/ethanol 9:1. The title compound is obtained as a solid; m.p. 244-246 °C; MS-ES$^+$: $(M+H)^+$ = 513; TLC $R_f$ (dichloromethane/ethanol 9:1) 0.46.

Example 1: ((*R*)-1-Phenyl-ethyl)-[6-(4-piperazin-1-ylmethyl-phenyl)-7*H*-pyrrolo[2,3-*d*]pyrimidin-4-yl]-amine

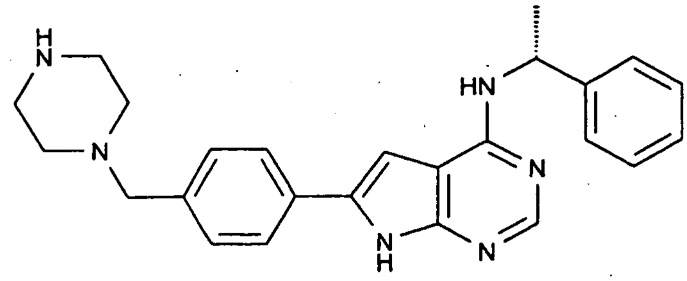

4-{4-[4-((*R*)-1-Phenyl-ethylamino)-7*H*-pyrrolo[2,3-*d*]pyrimidin-6-yl]-benzyl}-piperazine-1-carboxylic acid *tert*-butyl ester (1.8 g, 3.5 mmol) is dissolved in 150 mL of dioxane by gentle warming. To this solution is added a solution of 4 N hydrochloric acid in dioxane (Aldrich, Buchs, Switzerland) (5 mL, 20 mmol) and the mixture stirred at 50 to 60° C for 1 hour. The resulting suspension is diluted with 75 mL of methanol and stirred for 1 additional hour under reflux after which the mixture is cooled and the solvent evaporated. The residue is dissolved in diluted hydrochloric acid and washed with ethyl acetate. The aqueous phase is treated with solid potassium carbonate until basic and evaporated. The residue which contains inorganic salts is purified by flash chromatography using dichloromethane/methanol 7:3 containing 1% conc. ammonia. Pure fractions were taken up in ethyl acetate, washed with water and brine, dried with sodium sulfate and evaporated to give the title compound as a solid; m.p. 240-242 °C; MS-ES$^+$: $(M+H)^+$ = 413; TLC $R_f$ (dichloromethane/methanol 7:3 containing 1% conc. ammonia) 0.35; HPLC $t_R$ = 6.86 min.

Example 2: Dry-filled capsules

5000 capsules, each comprising as active ingredient 0.25 g of the compound of Example 1, are prepared as follows:

| Composition | |
|---|---|
| active ingredient | 1250 g |
| talcum | 180 g |
| wheat starch | 120 g |
| magnesium stearate | 80 g |
| lactose | 20 g |

Preparation process: The mentioned substances are pulverised and forced through a sieve of 0.6 mm mesh size. 0.33 g portions of the mixture are introduced into gelatin capsules using a capsule-filling machine.

Example 3: Soft capsules

5000 soft gelatin capsules, each comprising as active ingredient 0.05 g of the compound of Example 1, are prepared as follows:

| Composition | |
|---|---|
| active ingredient | 250 g |
| PEG 400 | 1 litre |
| Tween 80 | 1 litre |

Preparation process: The active ingredient is pulverised and suspended in PEG 400 (polyethylene glycol having an $M_r$ of from approx. 380 to approx. 420, Fluka, Switzerland) and Tween®80 (polyoxyethylene sorbitan monolaurate, Atlas Chem. Ind. Inc., USA, supplied by Fluka, Switzerland) and ground in a wet pulveriser to a particle size of approx. from 1 to 3 μm. 0.43 g portions of the mixture are then introduced into soft gelatin capsules using a capsule-filling machine.

## Claims

1. A compound of formula I

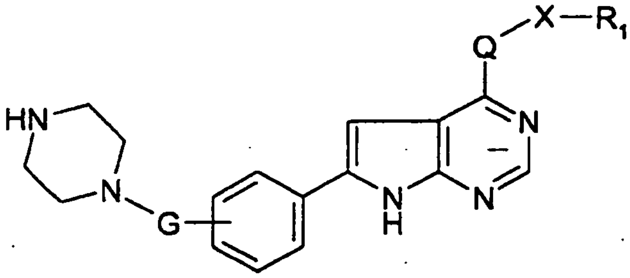

(I),

wherein

$R_1$ is phenyl, benzodioxolyl, pyridyl substituted by hydroxy or alkoxy having up to and including a maximum of 7 carbon atoms, indolyl substituted by halogen and alkyl having up to and including a maximum of 7 carbon atoms, or phenyl substituted by one or more radicals selected independently of one another from the group consisting of alkyl having up to and including a maximum of 7 carbon atoms, hydroxy, alkoxy having up to and including a maximum of 7 carbon atoms, halogen and benzyloxy;
G is $-CH_2-$ or -C(=O)-;
Q is -NH- or -O-, with the proviso that Q is -O- if G is -C(=O)-; and .
X is either not present, $-CH_2-$ or $-CH(CH_3)-$, with the proviso that substituted pyridyl or indolyl $R_1$ is bonded via a ring carbon atom if X is not present;

or a salt thereof.

2. A compound of formula I according to claim 1, wherein

$R_1$ is phenyl, benzodioxolyl, pyridyl substituted by hydroxy or alkoxy having up to and including a maximum of 7 carbon atoms, or phenyl substituted by one or more radicals selected independently of one another from the group consisting of alkyl having up to and including a maximum of 7 carbon atoms, hydroxy, alkoxy having up to and including a maximum of 7 carbon atoms, halogen and benzyloxy;
G is $-CH_2-$;
Q is -NH-; and
X is either not present, $-CH_2-$ or $-CH(CH_3)-$, with the proviso that substituted pyridyl $R_1$ is bonded via a ring carbon atom if X is not present;

or a salt thereof.

3. A compound of formula I according to claim 1 which is ((*R*)-1-phenyl-ethyl)-[6-(4-piperazin-1-ylmethyl-phenyl)-7*H*-pyrrolo[2,3-*d*]pyrimidin-4-yl]-amine or a pharmaceutically acceptable salt thereof.

4. A compound of formula I according to any one of claims 1 to 3, or a pharmaceutically acceptable salt thereof, for use in a method for the treatment of the human or animal body.

5. A pharmaceutical composition comprising a compound of formula I according to any one of claims 1 to 4, or a pharmaceutically acceptable salt thereof, together with at least one pharmaceutically acceptable carrier.

6. All. Use of a compound of formula I according to any one of claims 1 to 4, or a pharmaceutically acceptable salt thereof, for the preparation of a pharmaceutical composition for the treatment of a disease.

7. Use of a compound of formula I according to any one of claims 1 to 4, or a pharmaceutically acceptable salt thereof, for the preparation of a pharmaceutical composition for the treatment of a disease which responds to an inhibition of a protein tyrosine kinase.

**8.** A process for the preparation of a compound of formula I according to claim 1 or of a salt of such a compound, **characterized in that**

a) in order to prepare a compound of formula I, wherein G is $C_1$-$C_7$-alkylene, a compound of the formula II

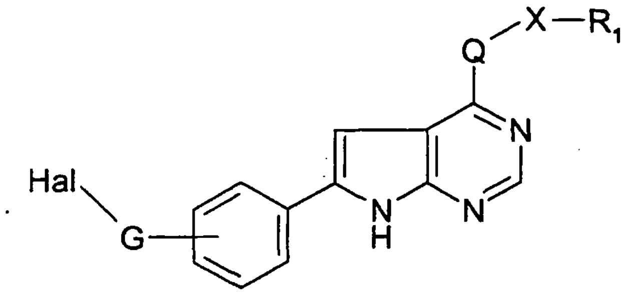

(II),

wherein Hal is halogen, G is $C_1$-$C_7$-alkylene and $R_1$, Q and X have the meanings as defined for a compound of formula I, is reacted with piperazine;

b) in order to prepare a compound of formula I, wherein G is -C(=O)- or $C_1$-$C_6$-alkylene-C(=O)- wherein the carbonyl group is attached to the piperazine moiety, a compound of formula III

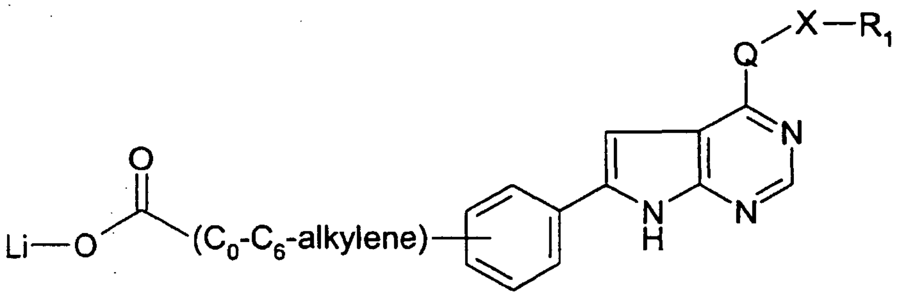

(III),

wherein the substituents and symbols have the meanings as defined for a compound of formula I, is reacted with piperazine; or

c) in order to prepare a compound of formula I, wherein G is $C_1$-$C_7$-alkylene, a compound of formula I, wherein G is -C(=O)- or $C_1$-$C_6$-alkylene-C(=O)- wherein the carbonyl group is attached to the piperazine moiety, is reacted with a reducing agent to produce the corresponding compound in which G is $C_1$-$C_7$-alkylene;

whereby functional groups which are present in the starting compounds of processes a) to c) and are not intended to take part in the reaction, are present in protected form if necessary, and protecting groups that are present are cleaved, whereby the said starting compounds may also exist in the form of salts provided that a salt-forming group is present and a reaction in salt form is possible;

and, if so desired, a compound of formula I thus obtained is converted into another compound of formula I, a free compound of formula I is converted into a salt, an obtained salt of a compound of formula I is converted into the free compound or another salt, and/or a mixture of isomeric compounds of formula I is separated into the individual isomers.

## Patentansprüche

**1.** Verbindung der Formel I

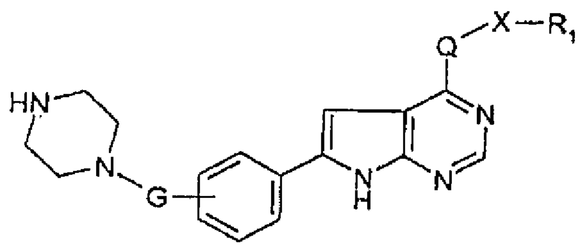

(I),

worin $R_1$ für Phenyl, Benzodioxolyl, Pyridyl, das durch Hydroxy oder Alkoxy mit bis zu und ehschließlich maximal 7 Kohlenstoffatomen substituiert ist, Indolyl, das durch Halogen und Alkyl mit bis zu und einschließlich maximal 7 Kohlenstoffatomen substituiert ist, oder Phenyl, das durch einen oder mehrere Reste substituiert ist, die unabhängig voneinander aus der Gruppe ausgewählt sind, die aus Alkyl mit bis zu und einschließlich maximal 7 Kohlenstoffatomen, Hydroxy, Alkoxy mit bis zu und einschließlich maximal 7 Kohlenstoffatomen, Halogen und Benzyloxy besteht, steht;
G für $-CH_2-$ oder -C(=O)- steht;
Q für -NH- oder -O- steht, wobei gilt, dass Q für -O- steht, wenn G für -C(=O)- steht und
X entweder nicht vorhanden ist, für $-CH_2-$ oder $-CH(CH_3)-$ steht, wobei gilt, dass substituiertes Pyridyl oder Indolyl von $R_1$ über ein Ringkohlenstoffatom gebunden ist, wenn X nicht vorhanden ist; oder ein Salz hiervon.

**2.** Verbindung der Formel I nach Anspruch 1, worin

$R_1$ für Phenyl, Benzodioxolyl, Pyridyl, das durch Hydroxy oder Alkoxy mit bis zu und einschließlich maximal 7 Kohlenstoffatomen substituiert ist, oder Phenyl, das durch einen oder mehrere Reste substituiert ist, die unabhängig voneinander aus der Gruppe ausgewählt sind, die aus Alkyl mit bis zu und einschließlich maximal 7 Kohlenstoffatomen, Hydroxy, Alkoxy mit bis zu und einschließlich maximal 7 Kohlenstoffatomen, Halogen und Benzyloxy besteht, steht;
G für $-CH_2-$ steht;
Q für -NH- steht und
X entweder nicht vorhanden ist, für $-CH_2-$ oder $-CH(CH_3)-$ steht, wobei gilt, dass substituiertes Pyridyl von $R_1$ über ein Ringkohlenstoffatom gebunden ist, wenn X nicht vorhanden ist;

oder ein Salz hiervon.

**3.** Verbindung der Formel I nach Anspruch 1, nämlich ((R)-1-Phenyl-ethyl)-[6-(4-piperazin-1-ylmethyl-phenyl)-7H-pyrrolo[2,3-d]pyrimidin-4-yl]-amin oder ein pharmazeutisch akzeptables Salz hiervon.

**4.** Verbindung der Formel I nach einem der Ansprüche 1 bis 3 oder ein pharmazeutisch akzeptables Salz hiervon zur Verwendung in einem Verfahren zur Behandlung des menschlichen oder tierischen Körpers.

**5.** Pharmazeutische Zusammensetzung, die eine Verbindung der Formel I nach einem der Ansprüche 1 bis 4 oder ein pharmazeutisch akzeptables Salz hiervon zusammen mit mindestens einem pharmazeutisch akzeptablen Träger umfasst.

**6.** Verwendung einer Verbindung der Formel I nach einem der Ansprüche 1 bis 4 oder eines pharmazeutisch akzeptablen Salzes hiervon zur Herstellung einer pharmazeutischen Zusammensetzung zur Behandlung einer Erkrankung.

**7.** Verwendung einer Verbindung der Formel I nach einem der Ansprüche 1 bis 4 oder eines pharmazeutisch akzeptablen Salzes hiervon zur Herstellung einer pharmazeutischen Zusammensetzung zur Behandlung einer Erkrankung, die auf eine Hemmung einer Proteintyrosinkinase reagiert.

**8.** Verfahren zur Herstellung einer Verbindung der Formel I nach Anspruch 1 oder eines Salzes einer derartigen Verbindung, **dadurch gekennzeichnet, dass**

a) zur Herstellung einer Verbindung der Formel I, worin G für $C_1$-$C_7$-Alkylen steht, eine Verbindung der Formel II

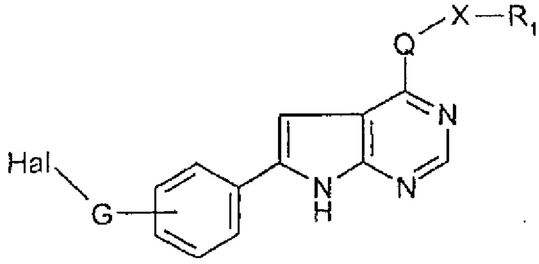

(II),

worin Hal für Halogen steht, G für $C_1$-$C_7$-Alkylen steht und $R_1$, Q und X die für eine Verbindung der Formel 1 angegebene Bedeutung besitzen, mit Piperazin umgesetzt wird;
b) zur Herstellung einer Verbindung der Formel I, worin G für -C(=O)- oder $C_1$-$C_6$-Alkylen-C(=O)- steht, worin die Carbonylgruppe an die Piperazineinheit gebunden ist, eine Verbindung der Formel III

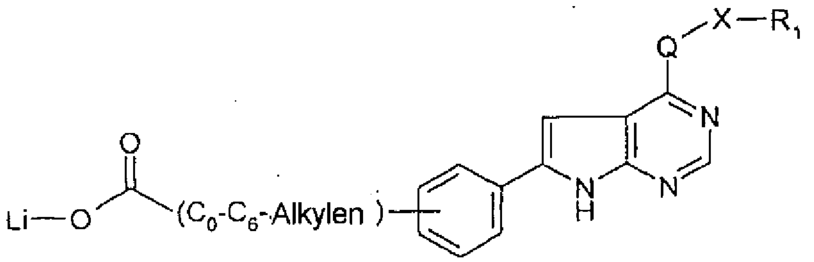

(III),

worin die Substituenten und Symbole die für eine Verbindung der Formel I angegeben Bedeutung besitzen, mit Piperazin umgesetzt wird, oder
c) zur Herstellung einer Verbindung der Formel I, worin G für $C_1$-$C_7$-Alkylen steht, eine Verbindung der Formel I, worin G für -C(=O)- oder $C_1$-$C_6$-Alkylen-C(=O)-steht, worin die Carbonylgruppe an die Piperazineinheit gebunden ist, mit einem Reduktionsmittel unter Bildung der entsprechenden Verbindung, worin G für $C_1$-$C_7$-Alkylen steht, umgesetzt wird;

wobei funktionelle Gruppen, die in den Ausgangsverbindungen der Verfahren a) bis c) vorhanden sind und die an der Reaktion nicht teilnehmen sollen, wenn nötig in geschützter Form vorhanden sind und Schutzgruppen, die vorhanden sind, abgespalten werden, wobei die Ausgangsverbindungen auch in Form von Salzen vorliegen können, vorausgesetzt, dass eine salzbildende Gruppe vorhanden ist, und eine Reaktion in Salzform möglich ist;
und, falls gewünscht, eine so erhaltene Verbindung der Formel I in eine weitere Verbindung der Formel I umgewandelt wird, eine freie Verbindung der Formel I in ein Salz umgewandelt wird, ein erhaltenes Salz einer Verbindung der Formel I in die freie Verbindung oder ein anderes Salz umgewandelt wird und/oder ein Gemisch von isomeren Verbindungen der Formel I in die individuellen Isomere getrennt wird.

## Revendications

**1.** Composé de formule I

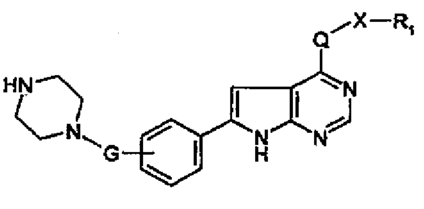

(I), I)

dans laquelle

$R_1$ représente un groupe phényle, benzodioxolyle, pyridyle substitué par un hydroxy ou un alcoxy ayant jusqu'à 7 atomes de carbone compris au plus, un indolyle substitué par un halogène et un alkyle ayant jusqu'à 7 atomes de carbone compris au plus, ou un phényle substitué par un ou plusieurs radicaux choisis indépendamment les uns des autres dans le groupe constitué d'un alkyle ayant jusqu'à 7 atomes de carbone compris au plus, d'un hydroxy, d'un alcoxy ayant jusqu'à 7 atomes de carbone compris au plus, d'un halogène et d'un benzyloxy ;
G représente $-CH_2-$ ou -C(=O)- ;
Q représente -NH- ou -O-, sous réserve que Q représente -O- si G représente -C(=O)- ; et
X, au choix, est absent ou représente $-CH_2-$ ou $-CH(CH_3)-$, sous réserve que le pyridyle ou l'indolyle substitué $R_1$ soit lié via un atome de carbone cyclique en l'absence de X ;

ou l'un de ses sels.

2. Composé de formule I selon la revendication 1, dans laquelle

$R_1$ représente un radical phényle, benzodioxolyle, pyridyle substitué par un hydroxy ou un alcoxy ayant jusqu'à 7 atomes de carbone compris au plus, ou un phényle substitué par un ou plusieurs radicaux choisis indépendamment les uns des autres dans le groupe constitué d'un alkyle ayant jusqu'à 7 atomes de carbone compris au plus, d'un hydroxy, d'un alcoxy ayant jusqu'à 7 atomes de carbone compris au plus, d'un halogène et d'un benzyloxy ;
G représente $-CH_2-$ ;
Q représente -NH- ; et
X, au choix, est absent ou représente $-CH_2-$ ou $-CH(CH_3)-$, sous réserve qu'un radical pyridyle substitué $R_1$ soit lié via un atome de carbone cyclique en l'absence de X ;

ou l'un de ses sels.

3. Composé de formule 1 selon la revendication 1, qui est une ((R)-1-phényl-éthyl)-[6-(4-pipérazin-1-ylméthyl-phényl)-7H-pyrrolo[2,3-d]pyrimidin-4-yl]-amine, ou l'un de ses sels pharmaceutiquement acceptables.

4. Composé de formule I selon l'une des revendications 1 à 3, ou l'un de ses sels pharmaceutiquement acceptables, destiné à être utilisé dans un procédé de traitement de l'organisme humain ou animal.

5. Composition pharmaceutique comprenant un composé de formule I selon l'une quelconque des revendications 1 à 4, ou l'un de ses sels pharmaceutiquement acceptables, ainsi qu'au moins un support acceptable sur le plan pharmaceutique.

6. Utilisation d'un composé de formule I selon l'une des revendications 1 à 4, ou de l'un de ses sels pharmaceutiquement acceptables, pour la préparation d'une composition pharmaceutique destinée au traitement d'une maladie.

7. Utilisation d'un composé de formule I selon l'une des revendications 1 à 4, ou de l'un de ses sels pharmaceutiquement acceptables, pour la préparation d'une composition pharmaceutique destinée au traitement d'une maladie qui réagit à l'inhibition d'une protéine tyrosine kinase.

8. Procédé de préparation d'un composé de formule I selon la revendication 1, ou de l'un de ses sels, **caractérisé en ce que**

a) pour préparer un composé de formule I, dans laquelle G représente un groupe alkylène en $C_1$ à $C_7$, on fait réagir avec une pipérazine un composé de formule II

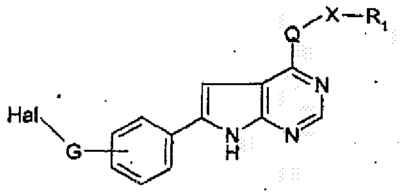

(II), I)

dans laquelle Hal représente un halogène, G représente un alkylène en $C_1$ à $C_7$, et $R_1$, Q et X sont tels que définis pour un composé de formule I ;

b) pour préparer un composé de formule I dans laquelle G représente - C(=O)- ou un groupe alkylène en $C_1$ à $C_6$-C(=O)-, où le groupe carbonyle est lié au groupement pipérazine, on fait réagir avec une pipérazine un composé de formule III

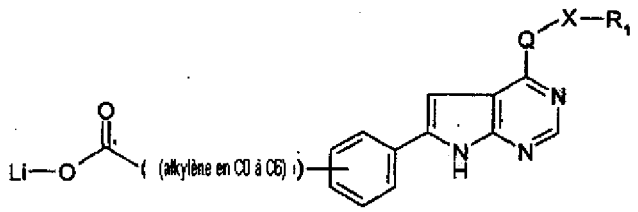

(III),

dans laquelle les substituants et les symboles sont tels que définis ci-avant pour un composé de formule I ; ou

c) pour préparer un composé de formule I dans laquelle G représente un groupe alkylène en $C_1$ à $C_7$, on fait réagir un composé de formule I dans laquelle G représente -C(=O)- ou un groupe alkylène en $C_1$ à $C_6$-C(=O)-, où le groupe carbonyle est lié au groupement pipérazine, avec un agent réducteur pour produire le composé correspondant dans lequel G représente un groupe alkylène en $C_1$ à $C_7$ ;

où les groupes fonctionnels présents dans les composés de départ des procédés a) à c) et qui ne sont pas destinés à participer à la réaction sont présents le cas échéant sous forme protégée, et les groupes protecteurs présents sont clivés, et où lesdits composés de départ peuvent également se présenter sous forme de sels, sous réserve qu'un groupe salificateur soit présent et qu'une réaction sous forme de sel soit possible ;

et où, si on le souhaite, un composé de formule I ainsi obtenu est transformé en un autre composé de formule I, un composé libre de formule I est transformé en un sel, un sel obtenu d'un composé de formule I est transformé en un composé libre correspondant ou en un autre sel, et/ou un mélange de composés isomères de formule I est séparé en isomères individuels.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US P6140332 P
- WO 03037897 A
- WO 0241882 A
- WO 03013541 A1
- EP 682027 A
- WO 9702266 A
- WO 9727199 A
- WO 9807726 A
- US 4659516 A
- WO 9917804 A
- US 5010099 A
- WO 9835958 A
- WO 0009495 A
- WO 0027820 A
- WO 0059509 A
- WO 9811223 A
- WO 0027819 A
- WO 0155114 A
- WO 0158899 A
- EP 0769947 A
- WO 0037502 A
- WO 9410202 A
- EP 0564409 A
- WO 9903854 A
- EP 0520722 A
- EP 0566226 A
- EP 0787722 A
- EP 0837063 A
- WO 9810767 A
- WO 9730034 A
- WO 9749688 A
- WO 9738983 A
- WO 9633980 A
- WO 9707131 A
- WO 9708193 A
- WO 9610028 A
- WO 9728161 A
- WO 9732879 A
- WO 9749706 A
- EP 0296110 A
- WO 0048571 A
- US 4100274 A
- US 5843901 A
- US 4636505 A


### Non-patent literature cited in the description


- **E. McGlynn et al.** *Europ. J. Biochem.,* 1992, vol. 207, 265-275
- **C. House et al.** *Europ. J. Biochem.,* 1984, vol. 140, 363-367
- **T. Akiyama et al.** *Science,* 1986, vol. 232, 1644
- **J. Wood et al.** *Cancer Res.,* 2000, vol. 60, 2178-2189
- **U. Trinks et al.** *J. Med. Chem.,* 1994, vol. 37 (7), 1015-1027
- **W. Lei et al.** *Anticancer Res.,* 1999, vol. 19 (1A), 221-228
- **T. Meyer et al.** *Int. J. Cancer,* 1989, vol. 43, 851
- **Santon, J.B. et al.** *Cancer Research,* 1986, vol. 46, 4701-4705
- **Ozawa, S. et al.** *Int. J. Cancer,* 1987, vol. 40, 706-710
- **Evans, B.D. et al.** *Brit. J. Cancer,* 1982, vol. 45, 466-8
- **N. Lydon et al.** *Oncogene Research,* 1990, vol. 5, 161-173
- **J. F. Geissler et al.** *Cancer Research,* 1992, vol. 52, 4492-4498
- Protective Groups in Organic Chemistry. Plenum Press, 1973
- **Houben-Weyl.** Methoden der organischen Chemie. Georg-Thieme-Verlag, 1974, vol. 15/1
- **Theodora W. Greene.** Protective Groups in Organic Synthesis. John Wiley & Sons, 1981
- **M. Prewett et al.** *Cancer Research,* 1999, vol. 59, 5209-5218
- **F. Yuan et al.** *Proc. Natl. Acad. Sci. USA,* December 1996, vol. 93, 14765-14770
- **Z. Zhu et al.** *Cancer Res.,* 1998, vol. 58, 3209-3214
- **J. Mordenti et al.** *Toxicologic Pathology,* 1999, vol. 27 (1), 14-21
- **M. S. O'Reilly et al.** *Cell,* 1994, vol. 79, 315-328
- **M. S. O'Reilly et al.** *Cell,* 1997, vol. 88, 277-285